# EUROPEAN PATENT SPECIFICATION

(11) **EP 2 821 053 B1**
(45) Date of publication and mention of the grant of the patent: **24.04.2019**
(21) Application number: 13754502.6
(22) Date of filing: 01.03.2013
(51) Int. Cl.: A61K 8/39, A61K 8/86, A61Q 19/10, A61K 8/36, C11D 1/04, A61K 31/00, A61K 33/00, C11D 1/06, C11D 3/20

(54) **SKIN CLEANSING COMPOSITION**
HAUTREINIGUNGSZUSAMMENSETZUNG
COMPOSITION NETTOYANTE POUR LA PEAU

(30) Priority: 02.03.2012 JP 2012047080
(43) Date of publication of application: 07.01.2015
(73) Proprietor: Kao Corporation, Chuo-Ku Tokyo 103-8210 (JP)
(72) Inventor: MASUI, Takashi, Tokyo 131-8501 (JP); TANAKA, Noriyuki, Tokyo 131-8501 (JP); ABE, Keita, Tokyo 131-8501 (JP)
(74) Representative: Hoffmann Eitle
(86) International application number: PCT/JP2013/055687
(87) International publication number: WO 2013/129653

(56) References cited:
- EP-A2- 0 690 044
- DE-A1-102004 027 323
- JP-A- H0 625 695
- JP-A- S6 197 395
- JP-A- H05 112 795
- JP-A- 2004 043 345
- JP-A- 2005 112 946
- JP-A- 2006 028 048
- JP-A- 2008 115 094
- JP-A- 2011 140 485
- JP-A- 2011 178 681
- 'Keshohin Handbook', 01 November 1996, NIKKO CHEMICALS CO., LTD. pages 163 - 165, XP008174613

## Description

### [Field of the Invention]

The present invention relates to a skin cleansing composition.

### [Background of the Invention]

It is known that fatty acid soap-based liquid cleansers can be obtained in the form of a liquid by incorporating a relatively large quantity of short-chain fatty acids and increasing the degree of neutralization. In contrast, it is known that fatty acid soap-based liquid cleansers can be obtained in the form of a paste by incorporating a relatively large quantity of long-chain fatty acids and lowering the degree of neutralization.

Cleansers in the form of a paste contain a relatively large quantity of long-chain fatty acids and have a low degree of neutralization, thereby achieving characteristics such as giving finer and more creamy foam and more pleasant feel, having better rinsing properties, and being less irritating to the skin compared to cleansers in the form of a liquid. Therefore, cleansers in the form of a paste are often used as face wash, and studies for enhancing foaming performance, rinsing performance, and skin-moisturizing properties after washing are ongoing (Patent Publications 1 and 2).

### [Citation List]

### [Patent Publication]

[Patent Publication 1] JP-A-2005-23069
[Patent Publication 2] JP-A-H07-508753

JP 2005-112946 is directed to a cleanser composition, which comprises the following components (A) to (D): (A) alkylpolyethercarboxylic acids or their salts; (B) an alkylpolyglycoside; (C) a fatty acid; (D) a polyoxyethylene fatty acid monoethanolamide.

JP 2011-140485 relates to a skin cleanser comprising a higher fatty acid or a salt thereof, a polyoxyethylene alkyl ether carboxylic acid or a salt thereof, an amphiphilic substance and water-insoluble particles.

JP 2006-028048 concerns a liquid detergent using both a polyoxyalkylene alkyl ether acetate and fatty acid soap. The gel-like or liquid detergent includes one or more kinds selected from a fatty acid N-alkylmonoethanol amide, a polyoxyethylene alkyl ether sulfate, an N-acylsarcosine salt and a fatty acid amide propylbetaine. It is said to have slight irritation to the skin and the eyes and excellent biodegradability, to be friendly to human and the environment, and to exhibit excellent detergency, foaming and foam qualities without drip in use.

DE 102004027323 relates to a cosmetic preparation (I), which comprises alkylethercarboxylate (a), ethoxylated glycerine (b), one or more soaps of 12-18C fatty acids (c) and water.

JP 2004-043345 concerns an extract of a plant belonging to the family Ophiolossaceae, which is said to have an excellent moisture-retaining effect and an excellent chapped skin-improving effect. A skin care preparation for the external use comprising the extract is also described.

EP 0690044 describes a process for producing an amidoethercarboxylic acid or a salt thereof, comprising reacting a fatty acid lower alkyl ester with ethanolamine or a derivative thereof, adding, if desired, ethylene oxide, and reacting the product with a monohalogenoacetic acid or a salt thereof in the presence of a solid alkali. Also disclosed is a surface active agent mixture containing the amidoether carboxylic acid or a salt thereof.

JP 2008-115094 is directed to a cosmetic contains 5-20 mass% of a fatty acid soap and 0.1-10 mass% of a polyoxyalkylene alkyl ether acetic acid surfactant of a specific formula.

### [Summary of the Invention]

The present invention relates to a skin cleansing composition, comprising the following components (A), (B), and (C):
(A) an alkyl ether carboxylic acid represented by the formula (1):

   R¹-O-(CH₂CH₂O)ₙ-CH₂-COOH (1)

   wherein, R¹ represents an alkyl group having 4 to 22 carbon atoms, and n represents a number of from 0 to 20,
   wherein, R¹ has an average carbon number of from 10.8 to 12.8,
   and wherein, the alkyl ether carboxylic acid contains a component in which n = 0 in an amount of from 4.3 to 30% by mass, and a component in which n = 1 and a component in which n = 2 in a total amount of 20% by mass or more and less than 40% by mass, based on the weight of component (A),
(B) a fatty acid represented by the formula (2):

   R²-COOH (2)

   wherein, R² represents a linear or branched alkyl group or alkenyl group having 9 to 21 carbon atoms, and (C) a neutralizer,
   wherein the total content of the components (A) and (B) is from 20 to 50% by mass, a degree of neutralization of the components (A) and (B) is from 0.6 to 0.9, and a viscosity at 30°C is from 10 to 50000 dPa·s.

### [Brief Description of the Drawing]

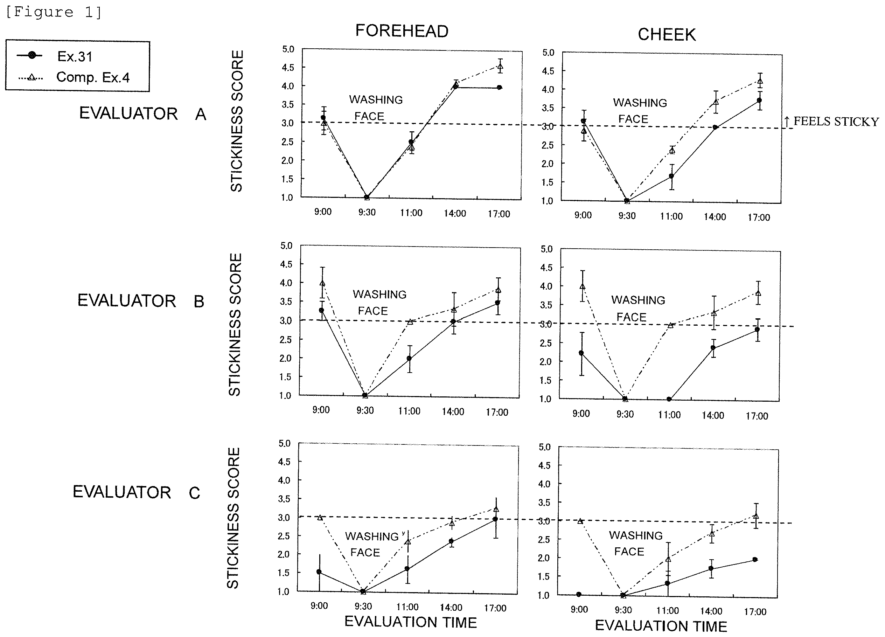

Figure 1 is a set of diagrams illustrating the evaluation results of stickiness over time in Test Example 1.

### [Detailed Description of the Invention]

General fatty acid soap-based cleansers in the form of a paste contain therein a large quantity of free fatty acids so that the degree of neutralization is kept low. While those free fatty acids serve to, for example, produce a creamy foam, they themselves do not act as a surfactant, and moreover, because they are an oil agent, they have problems such as creating some residual feel after washing (a feeling of something being left on the skin) and causing a reduction in detergency, and moreover, creating stickiness as time passes after washing the face.

The present invention relates to a skin cleansing composition which is excellent in the foam qualities, volume of foam, and rinsing properties while having high detergency and giving little residual feel on the skin after washing, and moreover, causing less stickiness even over time.

The present inventors have found that a skin cleansing composition which is excellent in the foam qualities, volume of foam, and rinsing properties while having high detergency and giving little residual feel on the skin after washing, and moreover, causing less stickiness even over time can be obtained by neutralizing a specific alkyl ether carboxylic acid and a specific fatty acid at a specific degree of neutralization.

The skin cleansing composition of the present invention is excellent in the foam qualities, volume of foam, and rinsing properties while having high detergency and giving little residual feel on the skin after washing. Further, when the skin cleansing composition of the present invention is prepared as face wash, the face feels less sticky even some time after washing the face compared to general soap paste face wash.

The alkyl ether carboxylic acid of the component (A) used in the present invention is represented by the formula (1).

In the formula, R¹ is an alkyl group having 4 to 22 carbon atoms, preferably an alkyl group having 6 to 20 carbon atoms, more preferably an alkyl group having 8 to 18 carbon atoms, even more preferably an alkyl group having 8 to 16 carbon atoms, and further preferably an alkyl group having 10 to 16 carbon atoms. Also, although the alkyl chain of R¹ may be either linear or branched, from the viewpoint of foaming properties, a linear alkyl group is preferred. Also, R¹ has an average carbon number of from 10.8 to 12.8, preferably from 10.8 to 12.5, and more preferably from 12.1 to 12.4. It is preferable that the average carbon number be within the above range since excellent foaming properties, foam qualities, and stability at low temperature are obtained.

Also, R¹ preferably contains two or more alkyl groups, and the content of a component having the alkyl chain length contained therein with the highest content is preferably 55% by mass or more and less than 97% by mass, more preferably from 60 to 95% by mass, and even more preferably from 70 to 95% by mass since excellent volume of foam and foam qualities are obtained.

Also, in the formula, n represents a number of from 0 to 20, and preferably from 0 to 12. It is to be noted that n represents the number of moles of ethylene oxide added, and the average number of moles of ethylene oxide added in the composition of the component (A) (an average value of n) is preferably from 1.5 to 10, more preferably from 2.5 to 4.5, much more preferably from 2.5 to 3.4, even more preferably from 2.8 to 3.4, and even more preferably from 2.8 to 3.1 since favorable foam qualities are achieved.

The alkyl ether carboxylic acid of the component (A) preferably contains, in the formula (1), a component in which n = 0 in an amount of from 4.3 to 30% by mass, preferably in an amount of from 4.9 to 27% by mass, more preferably in an amount of from 9.6 to 27% by mass, even more preferably from 9.9 to 16% by mass, and further preferably from 9.9 to 15% by mass. When the content of the component in which n = 0 is within the above range, the feeling of friction during rinsing is improved.

Further, the total content of a component in which n = 1 and a component in which n = 2 is 20% by mass or more and less than 40% by mass. From the viewpoint of foam qualities and the volume of foam, the total content of components in which n = 1 and 2 is preferably from 20 to 37% by mass, more preferably from 27 to 36.5% by mass, and even more preferably from 35 to 36.1% by mass.

In the formula (1), the alkyl ether carboxylic acid of the component (A) preferably has a mass ratio of the components in which n = 0, 1, 2, 3, and 4, in a component having an alkyl chain length of the highest content in the composition of R¹, (the mass of a component in which n = 0) : (the mass of a component in which n = 1) : (the mass of a component in which n = 2) : (the mass of a component in which n = 3) : (the mass of a component in which n = 4), of 1 : 0.99 to 3.50 : 0.89 to 3.00 : 0.76 to 3.00 : 0.63 to 1.6 from the viewpoint that foaming properties, detergency, and a feeling of friction during rinsing can be achieved simultaneously.

Also, in the formula (1), it is preferable that the content of a component in which n = 0 be 9.6% by mass or more and less than 12% by mass, and in a component having an alkyl chain length of the highest content in the composition of R¹, a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52, or the content of a component in which n = 0 be 12% by mass or more and 17% by mass or less and in a component having an alkyl chain length of the highest content in the composition of R¹, a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 0.99 from the viewpoint of foaming properties and rinsing properties.

Further, in the formula (1), it is preferable that the content of a component in which n = 0 be from 9.9 to 11.5% by mass and in a component having an alkyl chain length of the highest content in the composition of R¹, a ratio of (the mass of a component in which n = 0) : (the mass of a component in which n = 1) : (the mass of a component in which n = 2) : (the mass of a component in which n = 3) : (the mass of a component in which n = 4) = 1 : 1.58 to 1.84 : 1.72 to 2.17 : 1.49 to 2.00 : 1.14 to 1.52 or, in the formula (1), it is preferable that the content of a component in which n = 0 be from 13 to 17% by mass and in a component having an alkyl chain length of the highest content in the composition of R¹, a ratio of (the mass of a component in which n = 0) : (the mass of a component in which n = 1) : (the mass of a component in which n = 2) : (the mass of a component in which n = 3) : (the mass of a component in which n = 4) = 1 : 1.00 to 1.31 : 0.93 to 1.34 : 0.79 to 1.18 : 0.63 to 0.99 from the viewpoint of volume of foam, foam qualities, and rinsing properties.

It is preferable that, in the component (A), in the formula (1), R¹ be an alkyl group having 4 to 22 carbon atoms, R¹ has an average carbon number of from 10.8 to 12.8, and the content of a component having an alkyl chain length contained therein with the highest content be 55% by mass or more and less than 97% by mass, and further, n represents a number of from 0 to 20 and the average value thereof be from 1.5 to 10, and a component in which n = 0 be contained in an amount of from 4.9 to 27% by mass, and a component in which n = 1 and a component in which n = 2 be contained in a total amount of from 20 to 37% by mass.

It is preferable that, in the component (A), in the formula (1), R¹ be an alkyl group having 6 to 20 carbon atoms, R¹ has an average carbon number of 10.8 to 12.8, and the content of a component having the alkyl chain length contained therein with the highest content be 55% by mass or more and less than 97% by mass, and further, n represents a number of from 0 to 20 and the average value thereof be from 2.5 to 4.5, and a component in which n = 0 be contained in an amount of from 9.6 to 27% by mass, and a component in which n = 1 and a component in which n = 2 be contained in a total amount of from 27 to 36.5% by mass.

It is preferable that, in the component (A), in the formula (1), R¹ be an alkyl group having 8 to 18 carbon atoms, R¹ has an average carbon number of from 10.8 to 12.8, and the content of a component having the alkyl chain length contained therein with the highest content be 55% by mass or more and less than 97% by mass, and further, n represents a number of from 0 to 20 and the average value thereof be from 2.5 to 3.4, and a component in which n = 0 be contained in an amount of from 9.6 to 27% by mass, and a component in which n = 1 and a component in which n = 2 be contained in a total amount of from 27 to 36.5% by mass.

It is preferable that, in the component (A), in the formula (1), R¹ be an alkyl group having 8 to 16 carbon atoms, R¹ has an average carbon number of 10.8 to 12.5, and the content of a component having the alkyl chain length contained therein with the highest content be from 60 to 95% by mass, and further, n represents a number of from 0 to 20 and the average value thereof be from 2.8 to 3.4, and a component in which n = 0 be contained in an amount of from 9.6 to 27% by mass, preferably from 9.9 to 16% by mass, and a component in which n = 1 and a component in which n = 2 be contained in a total amount of from 27 to 36.5% by mass. A cleansing composition comprising the alkyl ether carboxylic acid having the aforementioned configuration can have improved volume of foam and foam qualities.

It is preferable that, in the component (A), in the formula (1), R¹ be an alkyl group having 10 to 16 carbon atoms, R¹ has an average carbon number of from 12.1 to 12.4, and the content of a component having the alkyl chain length which is contained in the highest content be from 60 to 95% by mass, and further, n represents a number of from 0 to 20 and the average value thereof be from 2.8 to 3.1, and a component in which n = 0 be contained in an amount of from 9.9 to 15% by mass, and the total content of a component in which n = 1 and a component in which n = 2 be from 35 to 36.1% by mass. A cleansing composition comprising the alkyl ether carboxylic acid having the aforementioned configuration can have improved volume of foam, foam qualities and less foam dripping.

It is to be noted that in the component (A) of the present invention, the distribution of the alkyl chain length of R¹, the average alkyl chain length of R¹, the amount of a component in which n = 0, the average number of added moles n, and a mass ratio of the components in which n = 0, 1, 2, 3, and 4 are obtained as follows from the gas chromatographic analysis of the alkyl ether carboxylic acid represented by the formula (1).

### [Distribution of the alkyl chain length of R¹]

From the peak areas obtained by gas chromatography, a peak area of each alkyl chain length corresponding to n = 0 mole was obtained, and setting the sum of the peak areas thus obtained at 100, the percentage of the distribution of each alkyl chain length was calculated. Similar calculation was carried out also as to n = 1 to 3 moles, and the percentage values of the distribution of each alkyl chain length corresponding to n = 0 to 3 moles were averaged out, whereby the distribution of the alkyl chain length of R¹ was obtained (from this, the alkyl group component contained in the highest content in the composition of R¹ can be specified).

### [Average alkyl chain length of R¹]

From the distribution of the alkyl chain length of R¹ obtained as above, the proportion of each component was obtained, which was multiplied by the number of carbon atoms of the corresponding alkyl chain length, and the resulting values were summed up. The value thus obtained was used as an average alkyl chain length.

### [Amount of a component in which n = 0, total content of a component in which n = 1 and a component in which n = 2]

In the composition of R¹, the alkyl chain length contained therein with the highest content was specified, and the peak areas of the component having alkyl chain length of the highest content corresponding to n = 0 to 10 were added up by gas chromatography. By setting the total amount thus obtained at 100%, the amount of a component in which n = 0, the total content of a component in which n = 1 and a component in which n = 2 were calculated.

### [Average number of added moles n]

In the composition of R¹, the alkyl chain length of the highest content was specified, and the peak areas of the component having the alkyl chain length of the highest content corresponding to n = 0 to 10 were added up by gas chromatography (the amount of a component in which n is 11 or more was so small that it was excluded from the calculation). By setting the total amount thus obtained at 1, each proportion of n = 0 to 10 was obtained. The resulting proportion was multiplied by each number of added moles, and the sum of the resulting values was used as the average number of added moles n.

### [Mass ratio of the components in which n = 0, 1, 2, 3, and 4]

As to the ratio of each of the components having different numbers of moles of EO added, the distribution of the alkyl chain length of R¹ was obtained from the peak area obtained by gas chromatography by the method described above, and the component having the alkyl chain length of the highest content in the composition of R¹ was specified, and the ratio of each of the components having different numbers of moles of EO added was specified by the area ratio of n = 0, n = 1, n = 2, n = 3, and n = 4 of the component having the alkyl chain length of the highest content.

The alkyl ether carboxylic acid of the component (A) has the aforementioned composition, and from the viewpoint of the lack of residual feel on the skin after washing, the content thereof is preferably 1% by mass or more, more preferably 2% by mass or more, even more preferably 5% by mass or more and preferably 30% by mass or less, more preferably 20% by mass or less, and even more preferably 10% by mass or less of the total composition. Further, the content of the component (A) is preferably from 1 to 30% by mass, more preferably from 2 to 20% by mass, and even more preferably from 5 to 10% by mass of the total composition.

The fatty acid of the component (B) used in the present invention is represented by the aforementioned formula (2).

In the formula, R² represents an alkyl group or an alkenyl group having 9 to 21 carbon atoms, which may be linear or branched. R² is preferably an alkyl group or an alkenyl group having 11 to 17 carbon atoms.

As the component (B), lauric acid, myristic acid, palmitic acid, stearic acid, or behenic acid is preferable.

Also, from the viewpoint of rinsing properties and the lack of residual feel on the skin after washing, in the fatty acid of the component (B), the content of a fatty acid in which R² has 15 to 21 carbon atoms is preferably from 20 to 80% by mass, more preferably from 40 to 75% by mass, and even more preferably from 50 to 70% by mass of the total fatty acid of the component (B).

The component (B) can be used alone or in combination of two or more thereof, and from the viewpoint of dissolution and disintegration of paste, the content thereof is preferably 15% by mass or more, more preferably 20% by mass or more, even more preferably 25% by mass or more and preferably 45% by mass or less, more preferably 40% by mass or less, and even more preferably 30% by mass or less of the total composition. Further, content of the component (B) is preferably from 15 to 45% by mass, more preferably from 20 to 40% by mass, and even more preferably from 25 to 30% by mass of the total composition.

The neutralizer of the component (C) used in the present invention is a compound exhibiting basicity, and examples thereof include sodium hydroxide, potassium hydroxide, triethanolamine, basic amino acids, and a mixture thereof. Among them, potassium hydroxide, triethanolamine, and basic amino acids are preferable, and from the viewpoint of dissolution and disintegration of paste, potassium hydroxide is preferable.

In the skin cleansing composition of the present invention, the degree of neutralization of the components (A) and (B) is from 0.6 to 0.9, preferably from 0.7 to 0.8. By adjusting the degree of neutralization within the above range, a favorable skin cleansing composition having high viscosity and excellent volume of foam and foam qualities, which leaves no residual feel on the skin after washing, is obtained.

According to the present invention, a skin cleansing composition which is excellent in the foam qualities, volume of foam, and rinsing properties while having high detergency and giving little residual feel on the skin after washing, and moreover, causing less stickiness even over time can be obtained by using the component (A) in combination with the component (B).

When only the component (A) is used, the skin cleansing composition cannot be obtained in the form of a paste, while when only the component (B) is used, residual feel is left on the skin after washing.

From the viewpoint of foam qualities, volume of foam, rinsing properties, and reducing residual feel on the skin after washing, the mass ratio of the component (A) to the component (B) is, preferably, as acid, (A) : (B) = 1 : 15 to 3 : 1, more preferably (A) : (B) = 1 : 10 to 2 : 1, and even more preferably (A) : (B) = 1 : 5 to 1 : 1 in the skin cleansing composition of the present invention.

Also, from the viewpoint of dissolution and disintegration of paste, the total content of the components (A) and (B) is 20% by mass or more, preferably 30% by mass or more, and 50% by mass or less, preferably 40% by mass or less of the total composition. Also, the total content of the components (A) and (B) is from 20 to 50% by mass, preferably from 30 to 40% by mass of the total composition.

The skin cleansing composition of the present invention can further comprise (D) a polyol, whereby the volume of foam, foam qualities, absence of a tight feeling, and the state of a paste can be further improved.

Examples of the polyol include glycerol, sorbitol, mannitol, erythritol, xylitol, maltitol, trehalose, sucrose, polyglycerol ethylene glycol, propylene glycol, 1,3-butylene glycol, diethylene glycol, dipropylene glycol, isoprene glycol, polyethylene glycol (an average molecular weight of 2000 or less), polypropylene glycol (an average molecular weight of 1500 or less), and diethylene glycol monoethyl ether.

Among them, from the viewpoint of dissolution and disintegration of paste and good handling properties, glycerol, sorbitol, propylene glycol, dipropylene glycol, and diethylene glycol monoethyl ether are preferable.

The polyol of the component (D) can be used alone or in combination of two or more thereof, and from the viewpoint of the volume of foam, foam qualities and dissolution and disintegration of paste, the content thereof is preferably 5% by mass or more, more preferably 10% by mass or more, even more preferably 20% by mass or more and preferably 50% by mass or less, more preferably 40% by mass or less, and even more preferably 30% by mass or less of the total composition. Further, the content of the component (D) is preferably from 5 to 50% by mass, more preferably from 10 to 40% by mass, and even more preferably from 20 to 30% by mass of the total composition.

The skin cleansing composition of the present invention can further comprise water as a solvent. The content of water is preferably from 10 to 70% by mass, more preferably from 15 to 60% by mass and even more preferably from 20 to 40% by mass of the total composition. Water is added as balance of the cleansing composition other than the aforementioned components and other components composing the cleansing composition.

The skin cleansing composition of the present invention may further comprise components used in ordinary cleansers such as surfactants other than the components (A) and (B), humectants other than the component (D), oil components, disinfecting agents, antiinflammatory agents, antiseptic agents, chelating agents, thickening agents, salts, pearlescent agents, scrub agents, fragrances, cooling agents, dyes, ultraviolet absorbers, antioxidants, and plant extracts.

The skin cleansing composition of the present invention is produced and obtained in the form of a paste by mixing the blending components in accordance with a common method.

At this point, the form of a paste means that the substance has a viscosity of from 10 to 50000 dPa·s at 30°C as measured by a B-type viscometer (manufactured by Tokyo Keiki Inc.). Further, from the viewpoint of easy spreading, dissolution, and disintegration of paste, the viscosity is preferably from 100 to 10000 dPa·s, and the viscosity is more preferably from 300 to 4000 dPa·s.

Also, in the skin cleansing composition of the present invention, the pH is preferably from 8 to 12, more preferably from 9 to 11. Also, the value of pH is measured in each cleansing composition diluted 20-fold with ion exchange water at 25°C.

The skin cleansing composition of the present invention is suitable as, for example, a face wash, a body soap, and a hand soap. The skin cleansing composition of the present invention is preferable as a face wash and a body soap.

The skin cleansing method using the skin cleansing composition of the present invention is exemplified as follows. That is, there is a method comprising applying an adequate amount of the skin cleansing composition of the present invention to the body, namely the body's skin areas such as face, hands, feet, and torso, lathering up and washing, and then rinsing off using warm water from a shower and the like. It is also possible to apply an adequate amount of the cleansing composition of the present invention to a washing aid such as a towel, a sponge, and a brush, and then lather up and wash.

In connection with the aforementioned embodiments, the present invention further discloses the following compositions, production method, and application.
<1> A skin cleansing composition, comprising the following components (A), (B), and (C):
   (A) an alkyl ether carboxylic acid represented by the formula (1):

      R¹-O-(CH₂CH₂O)ₙ-CH₂-COOH (1)

      wherein, R¹ represents an alkyl group having 4 to 22 carbon atoms, and n represents a number of from 0 to 20,
      wherein, R¹ has an average carbon number of from 10.8 to 12.8,
      and wherein, the alkyl ether carboxylic acid contains a component in which n = 0 in an amount of from 4.3 to 30% by mass, and a component in which n = 1 and a component in which n = 2 in a total amount of 20% by mass or more and less than 40% by mass,
   (B) a fatty acid represented by the formula (2):

      R²-COOH (2)

      wherein, R² represents a linear or branched alkyl group or alkenyl group having 9 to 21 carbon atoms, and
   (C) a neutralizer,
      wherein the total content of the components (A) and (B) is from 20 to 50% by mass, a degree of neutralization of the components (A) and (B) is from 0.6 to 0.9, and a viscosity at 30°C is from 10 to 50000 dPa·s.
<2> The skin cleansing composition according to the aforementioned <1>, wherein, in the component (A), in the formula (1), R¹ is preferably an alkyl group having 6 to 20 carbon atoms, more preferably an alkyl group having 8 to 18 carbon atoms, even more preferably an alkyl group having 8 to 16 carbon atoms, and further preferably an alkyl group having 10 to 16 carbon atoms.
<3> The skin cleansing composition according to the aforementioned <1> or <2>, wherein, in the component (A), in the formula (1), the average carbon number of R¹ is preferably from 10.8 to 12.5, and more preferably from 12.1 to 12.4.
<4> The skin cleansing composition according to any one of the aforementioned <1> to <3>, wherein, in the component (A), in the formula (1), the average value of n, the average number of moles added is preferably from 1.5 to 10, more preferably from 2.5 to 4.5, even more preferably from 2.5 to 3.4, even more preferably from 2.8 to 3.4, and further preferably from 2.8 to 3.1.
<5> The skin cleansing composition according to any one of the aforementioned <1> to <4>, wherein the component (A) contains, in the formula (1), a component in which n = 0 in an amount of preferably from 4.9 to 27% by mass, more preferably from 9.6 to 27% by mass, even more preferably from 9.9 to 16% by mass, and further preferably from 9.9 to 15% by mass.
<6> The skin cleansing composition according to any one of the aforementioned <1> to <5>, wherein, in the component (A), in the formula (1), the total content of a component in which n = 1 and a component in which n = 2 is preferably from 20 to 37% by mass, more preferably from 27 to 36.5% by mass, and even more preferably from 35 to 36.1% by mass.
<7> The skin cleansing composition according to any one of the aforementioned <1> to <6>, wherein, in the component (A), in the formula (1), R¹ is an alkyl group having 6 to 20 carbon atoms, the average value of n is from 2.5 to 4.5, and the content of a component in which n = 0 is from 9.6 to 27% by mass.
<8> The skin cleansing composition according to any one of the aforementioned <1> to <7>, wherein, in the component (A), in the formula (1), R¹ is an alkyl group having 8 to 18 carbon atoms, the average value of n is from 2.5 to 3.4, and the content of a component in which n = 0 is from 9.9 to 27% by mass.
<9> The skin cleansing composition according to any one of the aforementioned <1> to <8>, wherein, in the component (A), in the formula (1), R¹ is an alkyl group having 8 to 16 carbon atoms, the average value of n is from 2.8 to 3.4, the content of a component in which n = 0 is from 9.9 to 27% by mass, and the total content of a component in which n = 1 and a component in which n = 2 is from 27 to 36.5% by mass.
<10> The skin cleansing composition according to any one of the aforementioned <1> to <9>, wherein, in the component (A), in the formula (1), in a component having an alkyl chain length of the highest content in the composition of R¹, the mass ratio of the components in which n = 0, 1, 2, 3, and 4 is (the mass of a component in which n = 0) : (the mass of a component in which n = 1) : (the mass of a component in which n = 2) : (the mass of a component in which n = 3) : (the mass of a component in which n = 4) = 1 : 0.99 to 3.50 : 0.89 to 3.00 : 0.76 to 3.00 : 0.63 to 1.52.
<11> The skin cleansing composition according to any one of the aforementioned <1> to <10>, wherein, in the component (A), in the formula (1), R¹ contains two or more alkyl groups, and the content of a component having an alkyl chain length which is contained in the highest content is preferably 55% by mass or more and less than 97% by mass, more preferably from 60 to 95% by mass, even more preferably from 70 to 95% by mass.
<12> The skin cleansing composition according to any one of the aforementioned <1> to <11>, wherein, the component (A) contains, in the formula (1), a component in which n = 0 in an amount of 8% by mass or more and less than 12% by mass and has a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52 in a component having an alkyl chain length of the highest content in the composition of R¹, or contains a component in which n = 0 in an amount of from 12 to 17% by mass and has a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 0.99 in a component having an alkyl chain length of the highest content in the composition of R¹.
<13> The skin cleansing composition according to any one of the aforementioned <1> to <12>, wherein the content of the component (A) is preferably from 1 to 30% by mass, more preferably from 2 to 20% by mass, and even more preferably from 5 to 10% by mass of the total composition.
<14> The skin cleansing composition according to any one of the aforementioned <1> to <13>, wherein the component (B) is preferably lauric acid, myristic acid, palmitic acid, stearic acid, or behenic acid.
<15> The skin cleansing composition according to any one of the aforementioned <1> to <14>, wherein the component (B) preferably contains, in the formula (2), a fatty acid salt in which R² has 15 to 21 carbon atoms in an amount of preferably from 20 to 80% by mass, more preferably from 40 to 75% by mass, and even more preferably from 50 to 70% by mass.
<16> The skin cleansing composition according to any one of the aforementioned <1> to <15>, wherein the content of the component (B) is preferably from 15 to 45% by mass, more preferably from 20 to 40% by mass, and even more preferably from 25 to 30% by mass of the total composition.
<17> The skin cleansing composition according to any one of the aforementioned <1> to <16>, wherein the neutralizer of the component (C) is preferably potassium hydroxide, triethanolamine, or a basic amino acid, and more preferably potassium hydroxide.
<18> The skin cleansing composition according to any one of the aforementioned <1> to <17>, wherein the skin cleansing composition comprises a salt of the component (A) and the component (C) and a salt of the component (B) and the component (C), and a degree of neutralization of the components (A) and (B) is from 0.6 to 0.9, preferably from 0.7 to 0.8.
<19> The skin cleansing composition according to any one of the aforementioned <1> to <18>, wherein a mass ratio of the component (A) to the component (B) is preferably (A) : (B) = 1 : 15 to 3 : 1, more preferably (A) : (B) = 1 : 10 to 2 : 1, and even more preferably (A) : (B) = 1 : 5 to 1 : 1.
<20> The skin cleansing composition according to any one of the aforementioned <1> to <19>, wherein the total content of the components (A) and (B) is preferably from 30 to 40% by mass of the total composition.
<21> The skin cleansing composition according to any one of the aforementioned <1> to <20>, further comprising (D) a polyol in an amount of from 5 to 50% by mass.
<22> The skin cleansing composition according to the aforementioned <21>, wherein the polyol of the component (D) is preferably glycerol, sorbitol, propylene glycol, dipropylene glycol, or diethylene glycol monoethyl ether.
<23> The skin cleansing composition according to the aforementioned <21> or <22>, wherein the content of the component (D) is preferably from 10 to 40% by mass, and more preferably from 20 to 30% by mass of a total composition.
<24> The skin cleansing composition according to any one of the aforementioned <1> to <23>, wherein the content of water is preferably from 10 to 70% by mass, more preferably from 15 to 60% by mass, and even more preferably from 20 to 40% by mass of a total composition.
<25> A method for washing skin, comprising applying the skin cleansing composition according to any one of the aforementioned <1> to <24> to a skin part, followed by washing and then rinsing.
<26> Use of the composition according to any one of the aforementioned <1> to <24> as a skin cleanser.

### <Measurement Method>

In the present invention, the alkyl composition, the distribution of the moles of EO added, and the ratio of each component of the alkyl ether carboxylic acid were measured by the following analytical method with gas chromatography (GC). Unless otherwise noted, "%" represents % by mass.

### (GC analytical conditions)

GC instrument; the product of Agilent Technologies, 7890A
Column; the product of Agilent Technologies, DB-5 (30 m, an inner diameter of 0.25 mm, a film thickness of 0.25 µm)
Detector; FID
Carrier; helium gas, 1 mL/min
Conditions of temperature rising; temperature is raised at 5°C/min from 100°C to 325°C, and thereafter, maintained at 325°C for 35 minutes.

### (Method of sample pretreatment)

Into 50 mL of methanol, 150 mg of alkyl ether carboxylic acid was dissolved. Also, the cleansing composition was taken in an amount of 150 mg in terms of alkyl ether carboxylic acid equivalent and dissolved in 50 mL of methanol. Also, when the cleansing composition contained a strong anionic surfactant such as polyoxyethylene alkyl ether sulfate, the cleansing composition was collected in such an amount that the strong anionic surfactant was 250 mg or less. From these solutions, 1 mL was taken and applied to a solid phase cartridge (manufactured by Biotage Japan Ltd., Isolute SAX, 1 g, 3 mL, 500-0100-B) which had been conditioned with 4 mL of methanol in advance, and the filtrate was received in a 10 mL round-bottom test tube. Subsequently, , the eluate which was eluted with 6 mL of a solution of 4.6 g of formic acid in 100 mL of methanol was also collected in the same test tube. The solution thus collected was set in a block heater heated to 50°C, to which nitrogen gas was blown in, and the solution was concentrated to approximately 1 mL, which was then dried at room temperature by further blowing nitrogen gas. To the resulting product, 2 mL of a diazomethane-ether solution was added, and the resulting solution was left to stand at room temperature for 10 minutes while stirring to carry out derivatization. Subsequently, nitrogen gas was blown in at room temperature and the solution was concentrated to 500 µL or less, to which chloroform was then added to bring the total volume to 500 µL, and the resulting product was subjected to GC analysis.

It is to be noted that the diazomethane-ether solution was prepared by the following procedure using a diazomethane generator (manufactured by Miyamoto Riken Ind. Co., Ltd., GM-50). A first receiver and a second receiver, and the second receiver and a third receiver were connected using a silicone rubber plug and a Teflon (Registered trademark) tube. Into the second receiver, 0.8 g of N-methyl-N'-nitro-N-nitrosoguanidine was collected, to which 2.5 mL of ion exchange water was added. Into the third receiver, 10 mL of tert-butyl methyl ether was collected. The first, second, and third receivers were cooled on ice. Subsequently, the second receiver was fitted with a plastic syringe, into which 3 mL of a solution of 20 g of sodium hydroxide dissolved in 100 mL of ion exchange water was placed. This aqueous solution of sodium hydroxide was slowly added dropwise to generate diazomethane gas, and nitrogen gas was gently blown in from the first receiver side to dissolve the diazomethane gas in tert-butyl methyl ether in the third receiver, whereby a diazomethane-ether solution was obtained.

The following reagents were used in the aforementioned sample pretreatment.
Methanol (manufactured by Kanto Chemical Co., Inc., for high performance liquid chromatography, 25183-1B) Formic acid (manufactured by Wako Pure Chemical Industries, Ltd., special grade chemical, 066-00461) Chloroform (manufactured by Kanto Chemical Co., Inc., CICA first grade, 07278-01) N-Methyl-N'-nitro-N-nitrosoguanidine (manufactured by Kanto Chemical Co., Inc., CICA first grade, 25596-51) Tert-butyl methyl ether (manufactured by Kanto Chemical Co., Inc., CICA special grade, 04418-00)
Sodium hydroxide (manufactured by Wako Pure Chemical Industries, Ltd., special grade, 196-13761)

### <Production Example>

The alkyl ether carboxylic acid of the component (A) used in the skin cleansing composition of the present invention is obtainable by a production method of alkyl ether carboxylic acid, which comprises reacting ethylene oxide with one or two or more alcohols selected from alcohols having an alkyl group having 4 to 22 carbon atoms to obtain alkyl ethoxylate, and then allowing the alkyl ethoxylate thus obtained to undergo further reactions. Specifically, the alkyl ether carboxylic acid of the component (A) can be produced, for example, as follows. Also, unless otherwise noted, "%" represents % by mass.

### Production Example 1

Into a stainless steel autoclave with stirring and temperature controlling functions, 1144 g (6.14 mol) of lauryl alcohol [trade name: KALCOL 2098, manufactured by Kao Corporation], 60.2 g (0.281 mol) of myristyl alcohol [trade name: KALCOL 4098, manufactured by Kao Corporation], and 2.68 g (0.0478 mol) of potassium hydroxide were placed and dehydration was performed under reduced pressure. Subsequently, 996 g (22.6 mol) of ethylene oxide (EO) was introduced at 155°C and reactions were allowed to proceed at a reaction temperature of 155°C and a reaction pressure of 0.4 MPa for two hours. Upon completion of the reaction, the resulting mixture was stirred for 30 minutes at 80°C under a reduced pressure condition of 6 kPa. Then, after removing unreacted ethylene oxide, nitrogen was introduced to normalize the pressure, and 4.82 g (0.0482 mol) of 90% lactic acid was added into the autoclave, followed by stirring at 80°C for 30 minutes, whereby alkyl ethoxylate having 3.55 moles of EO added (hereinbelow, also referred to as "the produced AE") was obtained.

Into a glass reaction container with stirring and temperature controlling functions and an oxygen gas introduction tube, 90 g (0.2 mol) of the aforementioned product, 16.7 g of a 48% aqueous solution of sodium hydroxide (0.2 mol as sodium hydroxide), 0.9 g of a palladium-platinum-bismuth-based catalyst (activated carbon containing 4% of palladium, 1% of platinum, 5% of bismuth, and 50% of water content), and 494.4 g of water were each placed. While stirring, the liquid temperature was raised to 70°C, and while blowing oxygen in at a ratio of 27 mol% (with respect to the produced AE / hour), catalytic oxidation reactions were carried out at a reaction temperature of 70°C for 3.5 hours. The rate of reaction was 89%.

Upon completion of the reaction, the catalyst was filtered out from the reaction solution to provide an aqueous solution of sodium salt of alkyl ether carboxylic acid. Subsequently, 35% hydrochloric acid was added, and a liquid separation operation was performed to provide alkyl ether carboxylic acid, which will be referred to as EC1.

As a result of gas chromatography analysis, it was found that, in the formula (1), M = H, R¹ had lauryl group / myristyl group at a ratio of 95 / 5, the average carbon number was 12.1, and the average value of n was 2.8, and EC1 contained a component in which n = 0 in an amount of 14.7% by mass, and a component in which n = 1 and a component in which n = 2 in a total amount of 36.1% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.22 : 1.23 : 1.06 : 0.83.

### Production Example 2

According to Production Example 1, EO was reacted with a raw material containing a mixture of decyl alcohol [trade name: KALCOL 1098, manufactured by Kao Corporation], lauryl alcohol [trade name: KALCOL 2098, manufactured by Kao Corporation], myristyl alcohol [trade name: KALCOL 4098, manufactured by Kao Corporation], and cetyl alcohol [trade name: KALCOL 6098, manufactured by Kao Corporation] at a mass ratio of 10 / 70 / 15 / 5 to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ had decyl group/lauryl group/myristyl group/ palmityl group at a ratio of 10 / 70 / 15 / 5, the average carbon number was 12.3, and the average value of n was 3.3, and the alkyl ether carboxylic acid contained a component in which n = 0 in an amount of 15.2% by mass, and a component in which n = 1 and a component in which n = 2 in a total amount of 31.4% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.07 : 1.00 : 0.85 : 0.67.

### Production Example 3

Into a glass reaction container with stirring and temperature controlling functions, 372 g (2.00 mol) of lauryl alcohol was placed, and while stirring, the liquid temperature was raised to 70°C. Then, while adding 256 g (2.20 mol) of sodium monochloroacetate and 88 g (2.20 mol) of sodium hydroxide in divided portions, a reaction was allowed to proceed for five hours. Upon completion of the reaction, the precipitates were filtered out. Subsequently, 35% hydrochloric acid was added for acidification to obtain alkyl ether carboxylic acid (in the formula (1), M = H, R¹ was a lauryl group, and n = 0).

### Production Example 4

According to Production Example 1, EO was reacted with decyl alcohol as a raw material to obtain alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a decyl group, and the average value of n was 3.1, and the alkyl ether carboxylic acid contained a component in which n = 0 in an amount of 16% by mass, and a component in which n = 1 and a component in which n = 2 in a total amount of 37% by mass.

### Production Example 5

According to Production Example 1, EO was reacted with lauryl alcohol as a raw material to obtain alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a lauryl group, and the average value of n was 3.1, and the alkyl ether carboxylic acid contained a component in which n = 0 in an amount of 16% by mass, and a component in which n = 1 and a component in which n = 2 in a total amount of 37% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.19 : 1.13 : 0.94 : 1.

### Production Example 6

According to Production Example 1, EO was reacted with myristyl alcohol as a raw material to obtain alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a myristyl group, and the average value of n was 3.1, and the alkyl ether carboxylic acid contained a component in which n = 0 in an amount of 16% by mass, and a component in which n = 1 and a component in which n = 2 in a total amount of 37% by mass.

### Production Example 7

According to Production Example 1, EO was added to a raw material containing a mixture of lauryl alcohol and cetyl alcohol at a mass ratio of 20 / 80 to obtain alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ had lauryl group / palmityl group at a ratio of 20 / 80, and the average value of n was 3.1, and the alkyl ether carboxylic acid contained a component in which n = 0 in an amount of 16% by mass, and a component in which n = 1 and a component in which n = 2 in a total amount of 37% by mass.

### Production Example 8

According to Production Example 1, EO was reacted with lauryl alcohol as a raw material to obtain alkyl ethoxylate having 4.05 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a lauryl group, the average value of n was 3.5, and the resulting alkyl ether carboxylic acid contained a component in which n = 0 in an amount of 11.4% by mass, and a component in which n = 1 and a component in which n = 2 in a total amount of 30.6% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.31 : 1.38 : 1.25 : 1.06.

### Production Example 9

Into a stainless steel autoclave with stirring and temperature controlling functions, 1144 g (6.14 mol) of lauryl alcohol [trade name: KALCOL 2098, manufactured by Kao Corporation], 60.2 g (0.281 mol) of myristyl alcohol [trade name: KALCOL 4098, manufactured by Kao Corporation], and 2.6 g (0.0478 mol) of potassium hydroxide were placed and dehydration was performed under reduced pressure. Subsequently, 718 g (16.3 mol) of ethylene oxide (EO) was introduced at 155°C and a reaction was allowed to proceed at a reaction temperature of 155°C and a reaction pressure of 0.4 MPa for two hours. Upon completion of the reaction, the resulting mixture was cooled and then stirred for 30 minutes at 80°C under a reduced pressure condition of 6 kPa. Then, after removing unreacted ethylene oxide, nitrogen was introduced to normalize the pressure, and 4.82 g (0.0482 mol) of 90% lactic acid was added into the autoclave, followed by stirring at 80°C for 30 minutes, whereby alkyl ethoxylate having 2.55 moles of EO added was obtained.

Into a glass reaction container with stirring and temperature controlling functions, 600 g (2.00 mol) of the aforementioned product was placed, and while stirring, the liquid temperature was raised to 70°C. Then, while adding 256 g (2.20 mol) of sodium monochloroacetate and 88 g (2.20 mol) of sodium hydroxide in divided portions, a reaction was allowed to proceed for five hours. Upon completion of the reaction, 35% hydrochloric acid was added for acidification until pH was 2.8, and the resulting oil layer was collected to obtain alkyl ether carboxylic acid, which will be referred to as EC6.

As a result of gas chromatography analysis, it was found that, in the formula (1), M = H, R¹ had lauryl group / myristyl group at a ratio of 94 / 6, the average carbon number was 12.1, and the average value of n was 3.1, and EC6 contained a component in which n = 0 in an amount of 9.9% by mass, and a component in which n = 1 and a component in which n = 2 in a total amount of 35.4% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.65 : 1.92 : 1.74 : 1.32.

In the Examples, EC2 was obtained by mixing each of the alkyl ether carboxylic acids produced in Production Examples 5, 6, and 7 at a mass ratio of 78.75 / 15 / 6.25.

In the Examples, EC3 was obtained by mixing each of the alkyl ether carboxylic acid obtained in Production Examples 2 and 3 at a mass ratio of 90 / 10.

In the Examples, EC4 was obtained by mixing each of EC1 obtained in Production Example 1 and the alkyl ether carboxylic acid obtained in Production Example 4 at a mass ratio of 40 / 60.

In the Examples, EC5 was obtained by mixing each of the alkyl ether carboxylic acid obtained in Production Examples 2 and 8 at a mass ratio of 40 / 60.

### [Examples]

### Examples 1 to 30 and Comparative Examples 1 to 3

The skin cleansing compositions having the compositions as shown in Tables 3 to 7 were produced and the viscosity was measured. Also, the volume of foam, foam qualities, rinsing properties, lack of residual feel on the skin after washing, and lack of stickiness of the skin 6 hours after washing the face were evaluated. The results are shown in Tables 3 to 7 altogether.

Also, the composition of the component (A) used in Examples is as shown in Tables 1 and 2.

Also, for the average numbers of moles of EO added in commercially available alkyl ether carboxylic acids used in Examples and Comparative Examples (AKYPO RLM25 (manufactured by Kao Corporation), AKYPO RLM45 (manufactured by Kao Corporation), and AKYPO RLM100NV (manufactured by Kao Corporation)), values provided in the catalogue supplied by each vendor or values posted in the website of each vendor were referred to. Unknown alkyl composition, the amount of a component in which n = 0, and the total amount of a component in which n = 1 and a component in which n = 2 were analyzed by the aforementioned method.

### (Production method)

The components (A) and (B) were weighed out and placed in a beaker, and heated to 80°C to achieve complete melting. Subsequently, an 48% aqueous solution of KOH, an 48% aqueous solution of NaOH, triethanolamine, aminomethyl propanol, and arginine were added at 80°C in such amounts as to achieve a predetermined degree of neutralization. Further, water and polyol were added and the resulting mixture was stirred for 30 minutes, and then cooled to 30°C, whereby skin cleansing compositions were obtained in the form of a paste.

### (Evaluation method)

### (1) Viscosity:

Each skin cleansing composition was kept for 24 hours at 30°C after the production, and then the viscosity was measured under the following conditions.
Measurement apparatus: B-type viscometer (manufactured by Toki Sangyo Co., Ltd., model: TVB-10),
Measurement temperature: 30°C,
Rotor: T-D (8000 dPa·s or less) or T-E (8000 dPa·s or more),
Speed of rotation: 5 rpm,
Stand: Helical stand, and
Measurement time: 1 minute.

### (2) Face washing test:

The volume of foam, foam qualities, rinsing properties, and lack of residual feel on the skin after washing were evaluated by the following face washing test.

After moistening both hands with water (approximately 7 g), the skin cleansing composition (1 g) was placed on the palm. The paste was spread by rubbing both palms together, and after further adding approximately 10 g of water, foaming was continued for approximately 10 seconds. The face was massaged with the lather thus obtained and the volume of foam and foam qualities were evaluated. Subsequently, the face was rinsed with water and rinsing properties (speed of disappearance of slipperiness and adequate friction) were evaluated. Subsequently, water droplets on the face were wiped with a towel and the lack of residual feel on the skin after washing was evaluated by running fingers over the face. Each evaluation was conducted by one expert panelist in accordance with the following criteria.

### (2-1) Volume of foam;

5; Felt the volume of foam was very large.
4; Felt the volume of foam was large.
3; No opinion.
2; Felt the volume of foam was slightly small.
1; Felt the volume of foam was small.

### (2-2) Foam qualities;

5; Foam is very fine and soft.
4; Foam is slightly fine and soft.
3; Foam is soft.
2; Foam is slightly large, fragile, and slightly watery.
1; Foam is large, fragile, and watery.

### (2-3) Rinsing properties;

5; A slimy feel rapidly disappears, immediately followed by an appropriate feeling of friction.
4; A slimy feel slightly rapidly disappears, followed by an appropriate feeling of friction.
3; A slimy feel disappears, followed by a feeling of friction.
2; It takes some time for a slimy feel to disappear during rinsing, followed by a weak feeling of friction.
1; It took a long time for a slimy feel to disappear during rinsing, followed by a very weak feeling of friction.

### (2-4) Lack of residual feel on the skin after washing;

5; Residual feel (a slippery, unsmooth, or sticky feel) is completely absent.
4; Residual feel (a slippery, unsmooth, or sticky feel) is almost absent.
3; Residual feel (a slippery, unsmooth, or sticky feel) is slightly present.
2; Residual feel (a slippery, unsmooth, or sticky feel) is present.
1; Residual feel (a slippery, unsmooth, or sticky feel) is intensely present.

### (3) Lack of stickiness of the skin 6 hours after washing the face:

One expert panelist washed the face in a similar manner to (2) using each skin cleansing composition, and touched the skin (particularly, the forehead and cheek) with fingers 6 hours after washing the face to evaluate the lack of stickiness of the skin in accordance with the following criteria.
5; The skin virtually did not feel sticky.
4; The skin almost did not feel sticky.
3; The skin felt only a little sticky.
2; The skin felt sticky.
1; The skin strongly felt sticky.

**[Table 1]**

| | R1 (% by mass) | | | | Average carbon number | Average number of moles of EO added | n=0 Content ratio | n= 1, 2 Total amount |
|---|---|---|---|---|---|---|---|---|
| | C10 | C12 | C14 | C16 | | | | |
| EC1 | 0 | 95 | 5 | 0 | 12.1 | 2.8 | 14.7% | 36.1% |
| EC2 | 0 | 80 | 15 | 5 | 12.5 | 3.1 | 16.0% | 32.5% |
| EC3 | 8 | 73 | 13.5 | 4.5 | 12.2 | 2.82 | 27.0% | 27.1% |
| EC4 | 60 | 38 | 2 | 0 | 10.8 | 3.2 | 12.5% | 34.8% |
| EC5 | 4 | 88 | 6 | 2 | 12.1 | 3.4 | 13.3% | 31.0% |
| EC6 | 0 | 94 | 6 | 0 | 12.1 | 3.1 | 9.9% | 35.4% |

**[Table 2]**

| | R1(% by mass) | | | | Average carbon number | Average number of moles of EO added | n=0 Content ratio | n=1, 2 Total amount |
|---|---|---|---|---|---|---|---|---|
| | C10 | C12 | C14 | C16 | | | | |
| 25CA *1 | 0 | 68 | 26 | 6 | 12.8 | 2.5 | 16.0% | 32.5% |
| 45CA *2 | 0 | 68 | 26 | 6 | 12.8 | 4.5 | 9.6% | 31.2% |
| 100NV *3 | 0 | 68 | 26 | 6 | 12.8 | 10 | 4.9% | 20.4% |

| | | | | | | | | |
|---|---|---|---|---|---|---|---|---|
| *1: AKYPO RLM25 (manufactured by Kao Corporation) *2: AKYPO RLM45 (manufactured by Kao Corporation) *3: AKYPO RLM100NV (manufactured by Kao Corporation) | | | | | | | | |

**[Table 3]**

| Component (% by mass) | Example | | | | Comparative Example | | |
|---|---|---|---|---|---|---|---|
| | 1 | 2 | 3 | 4 | 1 | 2 | 3 |
| EC1 | 6.0 | 6.0 | 6.0 | 6.0 | 6.0 | 6.0 | 0 |
| Lauric acid | 2.2 | 2.2 | 2.2 | 2.2 | 2.2 | 2.2 | 2.2 |
| Myristic acid | 8.1 | 8.1 | 8.1 | 8.1 | 8.1 | 8.1 | 8.1 |
| Palmitic acid | 11.6 | 11.6 | 11.6 | 11.6 | 11.6 | 11.6 | 11.6 |
| Stearic acid | 8.1 | 8.1 | 8.1 | 8.1 | 8.1 | 8.1 | 8.1 |
| 48% Aqueous solution of potassium hydroxide | q.s. | q.s. | q.s. | q.s. | q.s. | q.s. | q.s. |
| Water | Balance | Balance | Balance | Balance | Balance | Balance | Balance |
| Total | 100 | 100 | 100 | 100 | 100 | 100 | 100 |
| Degree of neutralization | 0.8 | 0.7 | 0.6 | 0.9 | 0.5 | 1.0 | 0.8 |
| Total amount of fatty acids (B) | 30 | 30 | 30 | 30 | 30 | 30 | 30 |
| (A):(B) | 1:5 | 1:5 | 1:5 | 1:5 | 1:5 | 1:5 | 1:5 |
| Fatty acid with R2 = C15 to 21 (% by mass) | 66 | 66 | 66 | 66 | 66 | 66 | 66 |
| Viscosity (dPa·s) | 1000 | 1500 | 2000 | 500 | 3000 | 300 | 1500 |
| Volume of foam | 3 | 2.5 | 2 | 3.5 | 1.5 | 3 | 3 |
| Foam qualities | 3 | 3.5 | 3 | 2.5 | 2 | 2 | 3 |
| Rinsing properties | 3 | 3 | 2.5 | 2.5 | 2 | 2 | 3 |
| Lack of residual feel on the skin after washing | 3 | 2.5 | 2.5 | 3 | 1 | 2 | 1 |
| Lack of stickiness of the skin after 6 hours | 3 | 3 | 3 | 3 | 2 | 2 | 1 |

**[Table 4]**

| Component (% by mass) | Example | | | |
|---|---|---|---|---|
| | 5 | 6 | 7 | 8 |
| EC1 | 6.0 | 6.0 | 6.0 | 6.0 |
| Lauric acid | 2.2 | 2.2 | 2.2 | 2.2 |
| Myristic acid | 8.1 | 8.1 | 8.1 | 8.1 |
| Palmitic acid | 11.6 | 11.6 | 11.6 | 11.6 |
| Stearic acid | 8.1 | 8.1 | 8.1 | 8.1 |
| Glycerol | 10 | 20 | 30 | 40 |
| 48% Aqueous solution of potassium hydroxide | q.s. | q.s. | q.s. | q.s. |
| Water | Balance | Balance | Balance | Balance |
| Total | 100 | 100 | 100 | 100 |
| Degree of neutralization | 0.8 | 0.8 | 0.8 | 0.8 |
| Total amount of fatty acids(B) | 30 | 30 | 30 | 30 |
| (A):(B) | 1:5 | 1:5 | 1:5 | 1:5 |
| Fatty acid with R2 = C15 to 21 (% by mass) | 66 | 66 | 66 | 66 |
| Viscosity (dPa·s) | 1500 | 1500 | 1500 | 2000 |
| Volume of foam | 4 | 5 | 5 | 4 |
| Foam qualities | 4 | 4.5 | 5 | 5 |
| Rinsing properties | 4 | 5 | 4.5 | 4 |
| Lack of residual feel on the skin after washing | 4 | 5 | 4.5 | 4 |
| Lack of stickiness of the skin after 6 hours | 4 | 5 | 5 | 4 |

**[Table 5]**

| Component (% by mass) | Example | | | | | |
|---|---|---|---|---|---|---|
| | 9 | 10 | 11 | 12 | 13 | 14 |
| EC1 | 6.0 | 6.0 | 6.0 | 6.0 | 6.0 | 6.0 |
| Lauric acid | 3.5 | 2 | 3.9 | 2 | 4.8 | 2 |
| Myristic acid | 20.4 | 4 | 14.1 | 5.5 | 10.2 | 7 |
| Palmitic acid | 3.5 | 12 | 7.1 | 12 | 7.5 | 12 |
| Stearic acid | 2.6 | 12 | 4.9 | 10.5 | 7.5 | 9 |
| Glycerol | 30 | 30 | 30 | 30 | 30 | 30 |
| 48% Aqueous solution of potassium hydroxide | q.s. | q.s. | q.s. | q.s. | q.s. | q.s. |
| Water | Balance | Balance | Balance | Balance | Balance | Balance |
| Total | 100 | 100 | 100 | 100 | 100 | 100 |
| Degree of neutralization | 0.8 | 0.8 | 0.8 | 0.8 | 0.8 | 0.8 |
| Total amount of fatty acids(B) | 30.0 | 30.0 | 30.0 | 30.0 | 30.0 | 30.0 |
| (A):(B) | 1:5 | 1:5 | 1:5 | 1:5 | 1:5 | 1:5 |
| Fatty acid with R2 = C15 to 21 (% by mass) | 20 | 80 | 40 | 75 | 50 | 70 |
| Viscosity (dPa·s) | 700 | 3000 | 1500 | 2000 | 1500 | 2000 |
| Volume of foam | 5 | 3 | 5 | 3.5 | 5 | 3 |
| Foam qualities | 3 | 3 | 4 | 4 | 4.5 | 5 |
| Rinsing properties | 3 | 4 | 4 | 4 | 5 | 5 |
| Lack of residual feel on the skin after washing | 3 | 3 | 4 | 3.5 | 4 | 4 |
| Lack of stickiness of the skin after 6 hours | 3 | 3 | 4 | 3.5 | 5 | 4 |

**[Table 6]**

| Component (% by mass) | Example | | | | | | | |
|---|---|---|---|---|---|---|---|---|
| | 15 | 16 | 17 | 18 | 19 | 20 | 21 | 22 |
| EC6 | 6 | | | | | | | |
| EC2 | | 6 | | | | | | |
| EC3 | | | 6 | | | | | |
| EC4 | | | | 6 | | | | |
| EC5 | | | | | 6 | | | |
| 25CA | | | | | | 6 | | |
| 45CA | | | | | | | 6 | |
| 100NV | | | | | | | | 6 |
| Lauric acid | 2.2 | 2.2 | 2.2 | 2.2 | 2.2 | 2.2 | 2.2 | 2.2 |
| Myristic acid | 8.1 | 8.1 | 8.1 | 8.1 | 8.1 | 8.1 | 8.1 | 8.1 |
| Palmitic acid | 11.6 | 11.6 | 11.6 | 11.6 | 11.6 | 11.6 | 11.6 | 11.6 |
| Stearic acid | 8.1 | 8.1 | 8.1 | 8.1 | 8.1 | 8.1 | 8.1 | 8.1 |
| Glycerol | 30 | 30 | 30 | 30 | 30 | 30 | 30 | 30 |
| 48% Aqueous solution of potassium hydroxide | q.s. | q.s. | q.s. | q.s. | q.s. | q.s. | q.s. | q.s. |
| Water | Balance | Balance | Balance | Balance | Balance | Balance | Balance | Balance |
| Total | 100 | 100 | 100 | 100 | 100 | 100 | 100 | 100 |
| Degree of neutralization | 0.8 | 0.8 | 0.8 | 0.8 | 0.8 | 0.8 | 0.8 | 0.8 |
| Total amount of fatty acids(B) | 30.0 | 30.0 | 30.0 | 30.0 | 30.0 | 30.0 | 30.0 | 30.0 |
| (A):(B) | 1:5 | 1:5 | 1:5 | 1:5 | 1:5 | 1:5 | 1:5 | 1:5 |
| Fatty acid with R2 = C15 to 21 (% by mass) | 66 | 66 | 66 | 66 | 66 | 66 | 66 | 66 |
| Viscosity (dPa·s) | 1500 | 1500 | 1500 | 1500 | 1500 | 1500 | 1500 | 1500 |
| Volume of foam | 5 | 5 | 5 | 4 | 4 | 4 | 3.5 | 3 |
| Foam qualities | 5 | 5 | 4 | 3.5 | 4 | 3.5 | 3 | 3 |
| Rinsing properties | 4 | 4 | 5 | 4 | 4 | 3.5 | 3 | 2.5 |
| Lack of residual feel on the skin after washing | 5 | 4.5 | 4 | 4 | 4 | 3.5 | 3 | 3 |
| Lack of stickiness of the skin after 6 hours | 5 | 4.5 | 4 | 4 | 4 | 3.5 | 3 | 3 |

**[Table 7]**

| Component (% by mass) | Example | | | | | | | |
|---|---|---|---|---|---|---|---|---|
| | 23 | 24 | 25 | 26 | 27 | 28 | 29 | 30 |
| EC1 | 2 | 5 | 10 | 10 | 30 | 20 | 3 | 8 |
| Mixed fatty acids * | 20 | 25 | 30 | 20 | 15 | 20 | 45 | 40 |
| Glycerol | 30 | 30 | 30 | 30 | 30 | 30 | 30 | 30 |
| 48% Aqueous solution of potassium hydroxide | q.s. | q.s. | q.s. | q.s. | q.s. | q.s. | q.s. | q.s. |
| Water | Balance | Balance | Balance | Balance | Balance | Balance | Balance | Balance |
| Total | 100 | 100 | 100 | 100 | 100 | 100 | 100 | 100 |
| Degree of neutralization | 0.8 | 0.8 | 0.8 | 0.8 | 0.8 | 0.8 | 0.8 | 0.8 |
| Total amount of fatty acids(B) | 20.0 | 25.0 | 30.0 | 20.0 | 15.0 | 20.0 | 45.0 | 40.0 |
| (A):(B) | 1:10 | 1:5 | 1:3 | 1:2 | 2:1 | 1:1 | 1:15 | 1:15 |
| Fatty acid with R2 = C15 to 21 (% by mass) | 66 | 66 | 66 | 66 | 66 | 66 | 66 | 66 |
| Viscosity (dPa·s) | 500 | 1500 | 1500 | 1000 | 300 | 500 | 10000 | 5000 |
| Volume of foam | 4 | 4.5 | 4 | 3.5 | 3 | 3.5 | 5 | 4.5 |
| Foam qualities | 4 | 5 | 4 | 3.5 | 2.5 | 3.5 | 5 | 5 |
| Rinsing properties | 5 | 4.5 | 4 | 3.5 | 2.5 | 3 | 5 | 4.5 |
| Lack of residual feel on the skin after washing | 3.5 | 4.5 | 4 | 3.5 | 3 | 3.5 | 3 | 4.5 |
| Lack of stickiness of the skin after 6 hours | 3.5 | 5 | 4.5 | 4.5 | 4.5 | 4.5 | 3 | 5 |

| | | | | | | | | |
|---|---|---|---|---|---|---|---|---|
| *: 7% lauric acid, 27% myristic acid, 39% palmitic acid, and 27% stearic acid | | | | | | | | |

### Examples 31 to 36 and Comparative Example 4

The skin cleansing compositions having the compositions as shown in Tables 8 to 14 were produced in a similar manner to Examples 1 to 30.

The skin cleansing compositions of Examples 31 to 36 were excellent in the foam qualities, volume of foam, and rinsing properties while having high detergency and giving no residual feel on the skin after washing, and moreover, causing less stickiness even over time.

**[Table 8]**

| Example 31 | |
|---|---|
| Component | (% by mass) |
| 45CA | 8 |
| Palmitic acid(Palmac 505) | 24 |
| Glycerol | 15 |
| Propylene glycol | 5 |
| 48% Aqueous solution of potassium hydroxide | q.s. |
| Water | Balance |
| Total | 100 |
| Degree of neutralization | 0.8 |
| Viscosity (dPa·s) | 4000 |

**[Table 9]**

| Example 32 | |
|---|---|
| Component | (% by mass) |
| EC1 | 6.0 |
| Lauric acid | 2.7 |
| Myristic acid | 8.2 |
| Palmitic acid | 6.1 |
| Stearic acid | 7.5 |
| Behenic acid | 6.0 |
| Ethyldiglycol | 5.0 |
| Dipropylene glycol | 6.0 |
| Sorbitol | 12 |
| Glycerol | 8.6 |
| 48% Aqueous solution of potassium hydroxide | q.s. |
| Water | Balance |
| Total | 100 |
| Degree of neutralization | 0.8 |
| Viscosity (dPa·s) | 1500 |

**[Table 10]**

| Comparative Example 4 | |
|---|---|
| Component | (% by mass) |
| Lauric acid | 3.5 |
| Myristic acid | 20.4 |
| Palmitic acid | 3.5 |
| Stearic acid | 2.6 |
| Glycerol | 22 |
| Propylene glycol | 8 |
| 48% Aqueous solution of potassium hydroxide | q.s. |
| Water | Balance |
| Total | 100 |
| Degree of neutralization | 0.8 |
| Viscosity (dPa·s) | 2000 |

**[Table 11]**

| Example 33 | |
|---|---|
| Component | (% by mass) |
| EC1 | 6.0 |
| Lauric acid | 1.8 |
| Myristic acid | 5.6 |
| Palmitic acid | 11.6 |
| Stearic acid | 11.0 |
| Glycerol | 20 |
| Propylene glycol | 5 |
| Sorbitol | 5 |
| MERQUAT 550 (manufactured by Nalco Company) | 5 |
| 48% Aqueous solution of potassium hydroxide | q.s. |
| Water | Balance |
| Total | 100 |
| Degree of neutralization | 0.7 |
| Viscosity (dPa·s) | 1500 |

**[Table 12]**

| Example 34 | |
|---|---|
| Component | (% by mass) |
| EC1 | 6.0 |
| Lauric acid | 2.2 |
| Myristic acid | 8.1 |
| Palmitic acid | 11.6 |
| Stearic acid | 8.1 |
| Glycerol | 20 |
| 48% Aqueous solution of potassium hydroxide | q.s. |
| 48% Aqueous solution of sodium hydroxide | 0.5 |
| Water | Balance |
| Total | 100 |
| Degree of neutralization | 0.8 |
| Viscosity (dPa·s) | 2500 |

**[Table 13]**

| Example 35 | |
|---|---|
| Component | (% by mass) |
| EC1 | 6.0 |
| Lauric acid | 2.2 |
| Myristic acid | 8.1 |
| Palmitic acid | 11.6 |
| Stearic acid | 8.1 |
| Glycerol | 20 |
| 48% Aqueous solution of potassium hydroxide | q.s. |
| Triethanolamine | 0.5 |
| Aminomethyl propanol | 0.5 |
| Water | Balance |
| Total | 100 |
| Degree of neutralization | 0.8 |
| Viscosity (dPa·s) | 2500 |

**[Table 14]**

| Example 36 | |
|---|---|
| Component | (% by mass) |
| EC1 | 6.0 |
| Lauric acid | 2.2 |
| Myristic acid | 8.1 |
| Palmitic acid | 11.6 |
| Stearic acid | 8.1 |
| Glycerol | 20 |
| 48% Aqueous solution of potassium hydroxide | q.s. |
| Arginine | 0.5 |
| Water | Balance |
| Total | 100 |
| Degree of neutralization | 0.8 |
| Viscosity (dPa·s) | 2500 |

### Test Example 1

Using the skin cleansing compositions of Example 31 and Comparative Example 4, three evaluators evaluated stickiness over time.

First of all, stickiness of the face as of 9 a.m. was scored in accordance with the following criteria.
5; The face strongly feels sticky.
4; The face feels sticky.
3; The face feels a little sticky.
2; The face virtually does not feel sticky.
1; The face does not feel sticky at all.

Next, in a similar manner to the face washing test of (2), the face was washed at 9:30. Stickiness immediately after washing was also scored in a similar manner to the above.

Thereafter, scoring was performed in a similar manner to the above also at 11:00, 14:00, and 17:00.

The aforementioned evaluation was repeated for one week with respect to each cleansing composition, and the scores at each time point submitted by each evaluator were averaged out to obtain the average values. The results of plotting the values thus obtained against time are shown in Figure 1.

From the results of Figure 1, it was confirmed that compared to Comparative Example, the face washed with the skin cleansing composition of the present invention stayed less sticky even some time after washing the face.

## Claims

1. A skin cleansing composition, comprising the following components (A), (B), and (C):
(A) an alkyl ether carboxylic acid represented by the formula (1) :
R¹-O-(CH₂CH₂O)ₙ-CH₂-COOH (1)
wherein, R¹ represents an alkyl group having 4 to 22 carbon atoms, and n represents a number of from 0 to 20,
wherein, R¹ has an average carbon number of from 10.8 to 12.8,
and wherein, the alkyl ether carboxylic acid contains a component in which n = 0 in an amount of from 4.3 to 30% by mass, and a component in which n = 1 and a component in which n = 2 in a total amount of 20% by mass or more and less than 40% by mass, based on the weight of component (A),
(B) a fatty acid represented by the formula (2):
R²-COOH (2)
wherein, R² represents a linear or branched alkyl group or alkenyl group having 9 to 21 carbon atoms, and
(C) a neutralizer,
wherein the total content of the components (A) and (B) is from 20 to 50% by mass, a degree of neutralization of the components (A) and (B) is from 0.6 to 0.9, and a viscosity at 30°C is from 10 to 50000 dPa·s, as measured with a B-type viscometer.

2. The skin cleansing composition according to claim 1, further comprising (D) a polyol in an amount of from 5 to 50% by mass.

3. The skin cleansing composition according to claim 1 or 2, wherein the component (B) contains a fatty acid salt in which R² has 15 to 21 carbon atoms in an amount of from 20 to 80% by mass.

4. The skin cleansing composition according to any one of claims 1 to 3, wherein a mass ratio of the component (A) to the component (B), (A) : (B) is 1 : 10 to 2 : 1.

5. The skin cleansing composition according to any one of claims 1 to 4, wherein, in the component (A), in the formula (1), the average value of n, the average number of moles added, is from 1.5 to 10.

6. The skin cleansing composition according to any one of claims 1 to 5, wherein, in the component (A), in the formula (1), R¹ is an alkyl group having 6 to 20 carbon atoms, the average value of n is from 2.5 to 4.5, and the content of a component in which n = 0 is from 9.6 to 27% by mass.

7. The skin cleansing composition according to any one of claims 1 to 6, wherein, in the component (A), in the formula (1), R¹ is an alkyl group having 8 to 18 carbon atoms, the average value of n is from 2.5 to 3.4, and the content of a component in which n = 0 is from 9.9 to 27% by mass.

8. The skin cleansing composition according to any one of claims 1 to 7, wherein, in the component (A), in the formula (1), R¹ represents an alkyl group having 8 to 16 carbon atoms, the average value of n is from 2.8 to 3.4, the content of a component in which n = 0 is from 9.9 to 27% by mass, and the total content of a component in which n = 1 and a component in which n = 2 is from 27 to 36.5% by mass.

9. The skin cleansing composition according to any one of claims 1 to 8, wherein the component (A) has, in the formula (1), a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 3.50 : 0.89 to 3.00 : 0.76 to 3.00 : 0.63 to 1.52 in a component having an alkyl chain length of the highest content in the composition of R¹.

10. The skin cleansing composition according to any one of claims 1 to 9, wherein, in the component (A), in the formula (1), R¹ contains two or more alkyl groups, and the content of a component having an alkyl chain length which is contained in the highest content is 55% by mass or more and less than 97% by mass.

11. The skin cleansing composition according to any one of claims 1 to 10, which the component (A) contains, in the formula (1), a component in which n = 0 in an amount of 8% by mass or more and less than 12% by mass and has a ratio of (the mass of a component in which n = 0) : (the mass of a component in which n = 1) : (the mass of a component in which n = 2) : (the mass of a component in which n = 3) : (the mass of a component in which n = 4) of 1 : 1.53 to 1.87 : 1.59 to 2.25 : 1.33 to 2.16 : 1.14 to 1.52 in a component having an alkyl chain length of the highest content in the composition of R¹, or contains a component in which n = 0 in an amount of from 12 to 17% by mass and has a ratio of (the mass of a component in which n = 0) : (the mass of a component in which n = 1) : (the mass of a component in which n = 2) : (the mass of a component in which n = 3) : (the mass of a component in which n = 4) of 1 : 0.99 to 1.34 : 0.89 to 1.40 : 0.76 to 1.23 : 0.63 to 0.99 in a component having an alkyl chain length of the highest content in the composition of R¹.

12. A skin cleansing method, comprising applying the skin cleansing composition according to any one of claims 1 to 11 to a skin area, washing, and then rinsing.

13. Use of the composition according to any one of claims 1 to 11 as a skin cleanser.

## Patentansprüche

1. Hautreinigende Zusammensetzung, umfassend die folgenden Komponenten (A), (B) und (C):
(A) eine durch die Formel (1) dargestellte Alkylethercarbonsäure:
R¹-O-(CH₂CH₂O)ₙ-CH₂OH-COOH (1)
worin R¹ eine Alkylgruppe mit 4 bis 22 Kohlenstoffatomen darstellt und n eine Zahl von 0 bis 20 darstellt,
worin R¹ eine durchschnittliche Kohlenstoffanzahl von 10,8 bis 12,8 aufweist
und worin die Alkylethercarbonsäure eine Komponente, in der n = 0, in einem Gehalt von 4,3 bis 30 Masse-% und eine Komponente, in der n = 1 ist und eine Komponente, in der n = 2, in einem Gesamtgehalt von 20 Masse-% oder mehr und weniger als 40 Masse-% enthält, basierend auf dem Gewicht der Komponente (A),
(B) eine durch die Formel (2) dargestellte Fettsäure:
R²-COOH (2)
worin R² eine lineare oder verzweigte Alkylgruppe oder Alkenylgruppe mit 9 bis 21 Kohlenstoffatomen darstellt, und
(C) ein Neutralisierungsmittel,
worin der Gesamtgehalt der Komponenten (A) und (B) von 20 bis 50 Masse-% beträgt, der Neutralisationsgrad der Komponenten (A) und (B) von 0,6 bis 0,9 beträgt und die Viskosität bei 30°C von 10 bis 50.000 dPa·s beträgt, gemessen mit einem B-Typ-Viskosimeter.

2. Hautreinigende Zusammensetzung gemäß Anspruch 1, die weiterhin (D) ein Polyol in einem Gehalt von 5 bis 50 Masse-% umfasst.

3. Hautreinigende Zusammensetzung gemäß Anspruch 1 oder 2, worin die Komponente (B) ein Fettsäuresalz, in dem R² 15 bis 21 Kohlenstoffatome aufweist, in einem Gehalt von 20 bis 80 Masse-% enthält.

4. Hautreinigende Zusammensetzung gemäß einem der Ansprüche 1 bis 3, wobei das Masseverhältnis der Komponente (A) zur Komponente (B), (A) : (B) 1 : 10 bis 2 : 1 beträgt.

5. Hautreinigende Zusammensetzung gemäß einem der Ansprüche 1 bis 4, worin in der Komponente (A) in der Formel (1) der durchschnittliche Wert von n, die durchschnittliche addierte Molanzahl, von 1,5 bis 10 beträgt.

6. Hautreinigende Zusammensetzung gemäß einem der Ansprüche 1 bis 5, worin in der Komponente (A) in der Formel (1) R¹ eine Alkylgruppe mit 6 bis 20 Kohlenstoffatomen ist, der durchschnittliche Wert von n von 2,5 bis 4,5 beträgt und der Gehalt einer Komponente, in der n = 0, von 9,6 bis 27 Masse-% beträgt.

7. Hautreinigende Zusammensetzung gemäß einem der Ansprüche 1 bis 6, worin in der Komponente (A) in der Formel (1) R¹ eine Alkylgruppe mit 8 bis 18 Kohlenstoffatomen ist, der durchschnittliche Wert von n von 2,5 bis 3,4 beträgt und der Gehalt einer Komponente, in der n = 0, von 9,9 bis 27 Masse-% beträgt.

8. Hautreinigende Zusammensetzung gemäß einem der Ansprüche 1 bis 7, worin in der Komponente (A) in der Formel (1) R¹ eine Alkylgruppe mit 8 bis 16 Kohlenstoffatomen darstellt, der durchschnittliche Wert von n von 2,8 bis 3,4 beträgt, der Gehalt einer Komponente, in der n = 0, von 9,9 bis 27 Masse-% beträgt und der Gesamtgehalt einer Komponente, in der n = 1, und einer Komponente, in der n = 2, von 27 bis 36,5 Masse-% beträgt.

9. Hautreinigende Zusammensetzung gemäß einem der Ansprüche 1 bis 8, worin die Komponente (A) in der Formel (1) ein Verhältnis von (der Masse einer Komponente, in der n = 0) : (der Masse einer Komponente, in der n = 1) : (der Masse einer Komponente, in der n = 2) : (der Masse der Komponente, in der n = 3) : (der Masse der Komponente, in der n = 4) = 1 : 0,99 bis 3,50 : 0,89 bis 3,00 : 0,76 bis 3,00 : 0,63 bis 1,52 in einer Komponente aufweist, die einer Alkylkettenlänge des höchsten Gehalts in der Zusammensetzung von R¹ aufweist.

10. Hautreinigende Zusammensetzung gemäß einem der Ansprüche 1 bis 9, worin in der Komponente (A) in der Formel (1) R¹ zwei oder mehr Alkylgruppen enthält und der Gehalt einer Komponente mit einer Alkylkettenlänge, die im höchsten Gehalt enthalten ist, 55 Masse-% oder mehr und weniger als 97 % beträgt.

11. Hautreinigende Zusammensetzung gemäß einem der Ansprüche 1 bis 10, in welcher die Komponente (A) in der Formel (1) eine Komponente, in der n = 0, in einem Gehalt von 8 Masse-% oder mehr und weniger als 12 Masse-% enthält und ein Verhältnis von (der Masse einer Komponente, in der n = 0) : (der Masse einer Komponente, in der n = 1) : (der Masse einer Komponente, in der n = 2) : (der Masse der Komponente, in der n = 3) : (der Masse der Komponente, in der n = 4) von 1 : 1,53 bis 1,87 : 1,59 bis 2,25 : 1,33 bis 2,16 : 1,14 bis 1,52 in einer Komponente mit einer Alkylkettenlänge des höchsten Gehalts in der Zusammensetzung von R¹ aufweist, oder eine Komponente, in der n = 0, in einem Gehalt von 12 bis 17 Masse-% enthält und ein Verhältnis von (der Masse einer Komponente, in der n = 0) : (der Masse einer Komponente, in der n = 1) : (der Masse einer Komponente, in der n = 2) : (der Masse der Komponente, in der n = 3) : (der Masse der Komponente, in der n = 4) von 1 : 0,99 bis 1,34 : 0,89 bis 1,40 : 0,76 bis 1,23 : 0,63 bis 0,99 in einer Komponente mit einer Alkylkettenlänge des höchsten Gehalts in der Zusammensetzung von R¹ aufweist.

12. Hautreinigendes Verfahren, umfassend Applizieren der hautreinigenden Zusammensetzung gemäß einem der Ansprüche 1 bis 11 an einen Hautbereich, Waschen und dann Ausspülen.

13. Verwendung der Zusammensetzung gemäß einem der Ansprüche 1 bis 11 als Hautreiniger.

## Revendications

1. Composition nettoyante pour la peau, comprenant les composants (A), (B) et (C) suivants :
(A) un acide carboxylique d'éther alkylique représenté par la formule (1) :
R¹-O-(CH₂CH₂O)ₙ-CH₂-COOH (1)
dans laquelle, R¹ représente un groupe alkyle ayant de 4 à 22 atomes de carbone, et n représente un nombre de 0 à 20,
dans laquelle, R¹ a un nombre de carbone moyen de 10,8 à 12,8,
et dans laquelle, l'acide carboxylique d'éther alkylique contient un composant dans lequel n = 0 en une quantité de 4,3 à 30 % en masse, et un composant dans lequel n = 1 et un composant dans lequel n = 2 en une quantité totale de 20 % en masse ou plus et de moins de 40 % en masse, sur la base du poids du composant (A),
(B) un acide gras représenté par la formule (2) :
R²-COOH (2)
dans laquelle, R² représente un groupe alkyle linéaire ou ramifié ou un groupe alcényle ayant de 9 à 21 atomes de carbone, et
(C) un neutraliseur,
dans laquelle la teneur totale en les composants (A) et (B) est de 20 à 50 % en masse, un degré de neutralisation des composants (A) et (B) est de 0,6 à 0,9, et une viscosité à 30 °C est de 10 à 50 000 dPa.s, tel que mesuré avec un viscosimètre de type B.

2. Composition nettoyante pour la peau selon la revendication 1, comprenant en outre (D) un polyol en une quantité de 5 à 50 % en masse.

3. Composition nettoyante pour la peau selon la revendication 1 ou 2, dans laquelle le composant (B) contient un sel d'acide gras dans lequel R² a 15 à 21 atomes de carbone en une quantité de 20 à 80 % en masse.

4. Composition nettoyante pour la peau selon l'une quelconque des revendications 1 à 3, dans laquelle un rapport en masse du composant (A) sur le composant (B), (A)/(B) est de 1/10 à 2/1.

5. Composition nettoyante pour la peau selon l'une quelconque des revendications 1 à 4, dans laquelle, dans le composant (A), dans la formule (1), la valeur moyenne de n, le nombre moyen de moles ajoutées, est comprise entre 1,5 et 10.

6. Composition nettoyante pour la peau selon l'une quelconque des revendications 1 à 5, dans laquelle, dans le composant (A), dans la formule (1), R¹ est un groupe alkyle ayant de 6 à 20 atomes de carbone, la valeur moyenne de n est de 2,5 à 4,5, et la teneur d'un composant dans lequel n = 0 est de 9,6 à 27 % en masse.

7. Composition nettoyante pour la peau selon l'une quelconque des revendications 1 à 6, dans laquelle, dans le composant (A), dans la formule (1), R¹ est un groupe alkyle ayant de 8 à 18 atomes de carbone, la valeur moyenne de n est de 2,5 à 3,4, et la teneur d'un composant dans lequel n = 0 est de 9,9 à 27 % en masse.

8. Composition nettoyante pour la peau selon l'une quelconque des revendications 1 à 7, dans laquelle, dans le composant (A), dans la formule (1), R¹ représente un groupe alkyle ayant de 8 à 16 atomes de carbone, la valeur moyenne de n est de 2,8 à 3,4, la teneur d'un composant dans lequel n = 0 est de 9,9 à 27 % en masse, et la teneur totale d'un composant dans lequel n = 1 et un composant dans lequel n = 2 est comprise entre 27 et 36,5 % en masse.

9. Composition nettoyante pour la peau selon l'une quelconque des revendications 1 à 8, dans laquelle le composant (A) présente, dans la formule (1), un rapport de (la masse d'un composant dans lequel n = 0)/(la masse d'un composant dans lequel n = 1)/(la masse d'un composant dans lequel n = 2)/(la masse d'un composant dans lequel n = 3)/(la masse d'un composant dans lequel n = 4) = 1/0,99 à 3,50/0,89 à 3,00/0,76 à 3,00/0,63 à 1,52 dans un composant ayant une longueur de chaîne alkyle de la teneur la plus élevée dans la composition de R¹.

10. Composition nettoyante pour la peau selon l'une quelconque des revendications 1 à 9, dans laquelle, dans le composant (A), dans la formule (1), R¹ contient deux groupes alkyle ou plus, et la teneur d'un composant ayant une longueur de chaîne alkyle qui est contenu dans la teneur la plus élevée est de 55 % en masse ou plus et moins de 97 % en masse.

11. Composition nettoyante pour la peau selon l'une quelconque des revendications 1 à 10, dans laquelle le composant (A) contient, dans la formule (1), un composant dans lequel n = 0 en une quantité de 8 % en masse ou plus et moins de 12 % en masse et a un rapport de (la masse d'un composant dans lequel n = 0)/(la masse d'un composant dans lequel n = 1)/(la masse d'un composant dans lequel n = 2)/(la masse d'un composant dans lequel n = 3)/(la masse d'un composant dans lequel n = 4) de 1/1,53 à 1,87/1,59 à 2,25/1,33 à 2,16/1,14 à 1,52 dans un composant ayant une longueur de chaîne alkyle de la teneur la plus élevée dans la composition de R¹, ou contient un composant dans lequel n = 0 en une quantité de 12 à 17 % en masse et a un rapport de (la masse d'un composant dans lequel n = 0)/(la masse d'un composant dans lequel n = 1)/(la masse d'un composant dans lequel n = 2)/(la masse d'un composant dans lequel n = 3)/(la masse d'un composant dans lequel n = 4) de 1/0,99 à 1,34/0,89 à 1,40/0,76 à 1,23/0,63 à 0,99 dans un composant ayant une longueur de chaîne alkyle de la teneur la plus élevée dans la composition de R¹.

12. Procédé de nettoyage de la peau, comprenant l'application de la composition nettoyante pour la peau selon l'une quelconque des revendications 1 à 11 sur une surface de la peau, le lavage, puis le rinçage.

13. Utilisation de la composition selon l'une quelconque des revendications 1 à 11 en tant que nettoyant pour la peau.
